# EUROPÄISCHE PATENTANMELDUNG

(11) **EP 1 741 452 A2**
(43) Veröffentlichungstag der Anmeldung: **10.01.2007**
(21) Anmeldenummer: 06020357.7
(22) Anmeldetag: 18.03.2006
(51) Int. Cl.: A61L 9/01, B01D 53/34

(54) **Feste Geruchsabsorber bestehend aus anodischen Oxidschichten mit darin gespeichertem Aktivstoff**

(30) Priorität: 02.04.2005 DE 102005015209
(62) Teilanmeldung aus: 06005586.0
(71) Anmelder: Goldschmidt GmbH, 45127 Essen (DE)
(72) Erfinder: Kuhn, Hubert, 42697 Solingen (DE); Müller, Felix, 42555 Velbert (DE); Peggau, Jörg, 45357 Essen (DE); Thie, Gordon, 45527 Hattingen (DE)

(57) **Zusammenfassung**

Gegenstand der Erfindung sind feste Geruchsabsorber bestehend aus einem festen Träger mit nanostrukturierter Oberfläche und darin eingelagertem aktiven Zinkricinoleat als Geruchsabsorber.

## Beschreibung

Gegenstand der Erfindung sind feste Geruchsabsorber bestehend aus anodisch hergestellten Oxidschichten als Trägermaterial mit darin gespeicherten Aktivstoffen zur irreversiblen Bindung von geruchs- und schadstoffbelasteten Gasen.

Gerüche können aus den verschiedensten Quellen stammen. Manche sind natürlichen Ursprungs und werden von Menschen und Tieren exponiert, andere stammen aus landwirtschaftlichen oder industriellen Produktionsprozessen und können unmittelbar oder über die entsprechenden Produkte an die Umgebung abgegeben werden um dann wiederum in den Gegenständen des täglichen Gebrauchs wie Kleidung, Wäsche, Polstermöbel adsorbiert zu werden.

Kulturelle und ästhetische Standards haben ein Maß für die tolerierbaren Grenzen derartiger Gerüche gesetzt. Es sind daher im Stand der Technik eine Vielzahl von Mitteln und Verfahren zur Unterdrückung oder Entfernung von Gerüchen, insbesondere von unangenehm empfundenen Gerüchen, beschrieben.

Im Wesentlichen beschränken sich diese auf deren Maskierung durch andere stärkere aber und in der Regel als angenehm empfundene Gerüche oder deren irreversible Bindung durch chemische Reaktion oder physikalische Adsorption und Absorption an geeigneten Materialien.

Die Maskierung von Gerüchen ist eine wenig effektive Methode, da sie die Geruchsbildner nicht entfernt und daher immer nur eine kurzfristige erste Abhilfe sein kann.

Die irreversible Bindung der natürlichen und künstlichen unangenehmen Gerüche ist daher der effektivere Weg.

Weitverbreitete und für die verschiedenen Einsatzgebiete genutzte und als Geruchsabsorber verwendete Aktivstoffe sind Aktivkohle, Silikagel, Kieselgur, Fullererde, Zeolithe, Bentonite, Mineralien wie Clays und Montmorillonit. Weitere fixierende und abbauende Stoffe sind Cyclodextrine, Oxidationsmittel, Metallkatalysatoren (z.B. TiO₂), Enzyme, Mikroorganismen und biozide-/biostatische Verbindungen wie z.B. quaternäre Verbindungen (z.B. Biozide wie Benzalkonium-saccarinat, Cocoamido-amphopropionat, Alkylaminocarboxylat sowie auch Antistatika wie Cetyl-morpholinium-ethosulfat).

Daneben sind desodorierende Substanzen aus ein oder mehreren Metallsalzen einer unverzweigten oder verzweigten, ungesättigten oder gesättigten, ein- oder mehrfach hydroxylierten Fettsäure mit mindestens 16 Kohlenstoffatomen und/oder einer Harzsäure mit Ausnahme der Alkalimetallsalze sowie aus beliebigen Mischungen dieser Salze mit sich selbst, insbesondere Zinksalze in Kombinationen mit Zinksalzen der Abietinsäure oder mit Zinksalzen anderer gesättigter oder ungesättigter hydroxylierter Fettsäuren mit 16 und mehr C-Atomen sowie anderen oben angeführten Wirkstoffen und insbesondere Zinksalze von Fettsäuren, vorzugsweise der Ricinolsäure, deren Herstellung in der DE-B-17 92 074 beschrieben ist, allein oder in Kombination mit anderen Wirkstoffen, bekannte Geruchsabsorber.

Zinkricinoleat kann geruchsintensive organische Substanzen mit schwefel- bzw. stickstoffhaltigen funktionellen Gruppen, wie zum Beispiel Mercaptane, Thioether, niedermolekulare Carbonsäuren wie Isovalerinsäure oder auch Amine, chemisch binden.

Die Fähigkeit des Zinkricinoleats, Substanzen dieser Art chemisch fest zu binden, ermöglicht daher den Einsatz in industriellen und privaten Anwendungsbereichen zum Reduzieren von unangenehmen Haushalts- und Industriegerüchen.

Zinkricinoleat ist eine wachsartige Substanz, die mit Wasser aktiviert werden muss, um in der Lage zu sein Gerüche zu binden. Da es aber in Wasser weitestgehend unlöslich ist, muss es, um wirksame Zubereitungen zu erhalten, daher in Kombination mit Lösungsmitteln und Lösungsvermittlern eingesetzt werden. Als Lösungsmittel werden meistens ein-oder mehrwertige Alkohole, gegebenenfalls unter Zusatz von Wasser, verwendet. Üblicherweise eingesetzte hochethoxylierte Lösungsvermittler vermögen auch in hohen Konzentrationen das Zinkricinoleat allein nicht in Lösung zu halten, und es werden dadurch auch keine fließfähigen Produkte erhalten.

Als Lösungsvermittler werden Partialester von Di- oder Polyhydroxyalkanen, Mono- und Disacchariden, Polyethylenglykolen oder Alkanolaminen mit den En-Addukten von Maleinsäureanhydrid an mindestens einfach ungesättigten Carbonsäuren mit einer Kettenlänge von 10 bis 25 Kohlenstoffatomen und Säurezahlen von 10 bis 140 beschrieben, welche vorzugsweise mit Amino- und/oder Amidoverbindungen wie Triethanolamin oder Glykolester der Asparagin- und der Glutaminsäure durch Bildung salzartiger Bindungen auf pH-Werte um 6,5 gepuffert werden.

Zubereitungen mit diesen Lösungsvermittlern waren jedoch nicht fließfähig, und die daraus formulierten Deodorantlösungen neigen bereits bei sehr geringen Wassergehalten zur Trübung und Ausfällung einzelner Komponenten (vergl. DE-A-40 14 055, Seite 2, Zeilen 50-52).

Die Anstrengungen im Stand der Technik konzentrierten sich daher darauf, verbesserte Lösungen des Zinkricinoleats bereitzustellen. Die heute bereitgestellten Formulierungen stellen ein komplexes Gemisch aus den verschiedensten Bestandteilen dar, in dem die eigentliche Wirksubstanz nur noch in geringen Konzentrationen vorhanden ist.

Bisherige Versuche, einen Geruchsabsorber auf Substraten zu binden, führten zwar zu einer leichten Geruchsverminderung, jedoch zeigten alle Versuche nicht die gewünschte Effektivität. Da das Zinkricinoleat hier unaktiviert bzw. komplexiert auf den Substraten vorliegt, ist nur ein geringer Anteil geruchsabsorbierender Wirkung nutzbar.

Die einzigen kommerziell umgesetzten Geruchsabsorber-Produkte sind wässrige Systeme, die über spezielle Aktivierungen eine deutlich bessere Geruchsauslöschung gewährleisten. Wie in nachfolgenden Veröffentlichungen nachzulesen ist:
Müller F., J. Peggau, H. Kuhn, Investigations on Zinc Ricinoleate as odour absorber with molecular dynamics calculations, Jorn. Com. Esp. Deterg. 30 (2000) 83-91.
Kuhn H., F. Müller, J. Peggau, R. Zekorn, Mechanism of the Odor-Absorption Effect of Zinc Ricinoleate. A Molecular Dynamics Computer Simulation, J. Surf. Deterg. 3 (2000) 335-343.
Müller F., J. Peggau, T. Böhmer, New results on odor absorbtion with Zinc Ricinoleate, 41. wfk International Detergency Conference, Proceedings (2003) 130-137.

In vielen Anwendungen können jedoch keine wässrigen Systeme eingesetzt werden, wie z.B. in Bereichen mit sehr tiefen bzw. sehr hohen Temperaturen. Zudem kommt es bei wässrigen Systemen immer zu Abwasserproblemen, wie z.B. in hoch verdünnten Systemen ein starkes Wachstum von Mikroorganismen zu beobachten ist. Einige Substrate, wie z.B. Schichtsilikate, zeigen ein komplexierendes Verhalten, so dass das Zink aus dem Zinkricinoleat fest mit dem Substrat verbunden bleibt und für eine Reaktion mit Geruchs- bzw. Schadstoffen nicht mehr zur Verfügung steht.

Ähnlich problematisch sind die Bindungsverhältnisse zwischen Zinkricinoleat und unstrukturierten d.h. nicht nanostrukturierten Substraten, an die das Zinkricinoleat so fest gebunden vorliegt, dass es für den vorgesehenen Anwendungszweck inaktiv ist.

Eine Aufgabe der vorliegenden Erfindung bestand daher darin, einen einfachen Geruchsabsorber bereitzustellen, der in der Lage ist, sowohl bereits vorhandene als auch neu entstehende Gerüche schnell und dauerhaft zu beseitigen.

Diese Aufgabe wird gelöst durch nanostrukturierte Oberflächen als Träger und darin eingelagertem aktiven Zinkricinoleat als Geruchsabsorber.

Ein Gegenstand der Erfindung sind daher feste Geruchsabsorber bestehend aus einem festen Träger mit nanostrukturierter Oberfläche und darin eingelagertem aktiven Zinkricinoleat als Geruchsabsorber.

Ein weiterer Gegenstand der Erfindung sind daher feste Geruchsabsorber, welche dadurch gekennzeichnet sind, dass als festes Trägermaterial nanoporöse Materialien verwendet werden die fähig sind, Wasser an der Oberfläche zu fixieren.

Ein weiterer Gegenstand der Erfindung sind daher feste Geruchsabsorber, welche dadurch gekennzeichnet sind, dass als festes Trägermaterial nanoporöse Metalloxide verwendet werden.

Ein weiterer Gegenstand der Erfindung sind daher feste Geruchsabsorber, welche dadurch gekennzeichnet sind, dass als festes Trägermaterial nanoporöse anodisch oxidierte Metalle verwendet werden.

Ein weiterer Gegenstand der Erfindung sind daher feste Geruchsabsorber, welche dadurch gekennzeichnet sind, dass als festes Trägermaterial nanoporöse anodisch oxidierte Metalle, ausgesucht aus der Gruppe Cr, Hf, Nb, V, Ta, Zr, verwendet werden.

Ein weiterer Gegenstand der Erfindung sind daher feste Geruchsabsorber, welche dadurch gekennzeichnet sind, dass als festes Trägermaterial nanoporöses anodisch oxidiertes Aluminium oder nanostrukturiertes Titandioxid verwendet wird.

Ein weiterer Gegenstand der Erfindung sind daher feste Geruchsabsorber, welche dadurch gekennzeichnet sind, dass als Aktivstoff mindestens eine Verbindung ausgesucht aus der Gruppe der aktiven Geruchsabsorber, insbesondere ein oder mehreren Metallsalzen einer unverzweigten oder verzweigten, ungesättigten oder gesättigten, ein oder mehrfach hydroxylierten Fettsäure mit mindestens 16 Kohlenstoffatomen und/oder einer Harzsäure mit Ausnahme der Alkalimetallsalze sowie aus beliebigen Mischungen dieser Salze mit sich selbst oder weiteren Wirkstoffen, verwendet wird.

Ein weiterer Gegenstand der Erfindung sind daher feste Geruchsabsorber, welche dadurch gekennzeichnet sind, dass Zinksalze in Kombinationen mit Zinksalzen der Abietinsäure oder mit Zinksalzen anderer gesättigter oder ungesättigter hydroxylierter Fettsäuren mit 16 und mehr C-Atomen sowie anderen oben angeführten Wirkstoffen eingesetzt werden und insbesondere Zinksalze von Fettsäuren, vorzugsweise das Zinksalz der Ricinolsäure.

Ein weiterer Gegenstand der Erfindung sind daher feste Geruchsabsorber, welche dadurch gekennzeichnet sind, dass der aktive Geruchsabsorber mit bekannten Solubilisatoren und/oder Aktivatoren, insbesondere einem oder mehreren anionischen Tensiden, Aminosäuren, insbesondere Arginin und Lysin, und/oder mit weiteren desodorierenden Wirkstoffen, wie quaternären Ammoniumverbindungen oder Cyclodextrinen, kombiniert wird.

Ein weiterer Gegenstand der Erfindung ist daher die Verwendung der festen Geruchsabsorber zur Desodorierung von geruchs- oder schadstoffbelasteten Gasen.

Bei nanostrukturiertem Titandioxid und nanostrukturiertem Aluminiumoxid, dessen Oxidschicht durch anodische Oxidation hergestellt wird, findet zwar auch eine feste Bindung bzw. Einlagerung von Zinkricinoleat statt, jedoch überraschenderweise ohne dass es dadurch inaktiviert wird und ohne zusätzliches Wasser, Lösungsvermittler und den weiteren im Stand der Technik erforderlichen Hilfs- und Zusatzstoffen.

Die daraus resultierende Depotwirkung dieser Substrate ermöglicht die Anwendung von Zinkricinoleat neben den klassischen Anwendungen daher auch in Bereichen, in denen durch hohe oder tiefe Temperaturen die Anwendung von wässrigen Systemen nicht möglich ist.

Durch die Depotwirkung werden Geruchs- und Schadstoffe fest an das Substrat gebunden und können gezielt entsorgt werden. Abwasserprobleme bzw. das Wachstum von Mikroorganismen kann hierdurch unterbunden werden. Durch die Aktivierung des Zinkricinoleats auf den o. g. Substraten und die damit im Vergleich zu unaktiviertem Zinkricinoleat höheren geruchabsorbierenden Wirkung, ist ein kommerzieller Einsatz von diesen festen Geruchsabsorbern möglich.

Anodische Oxidschichten und die Verfahren zu ihrer Herstellung gehören zum bekannten Stand der Technik.

Zur Herstellung dieser erfindungsgemäß verwendeten Oxidschichten sind alle Metalle, die mit den anodisch gebildeten Oxiden fest haftende poröse Schichten geeigneter Schichtdicke und Porösität bilden und die unter Anwendungsbedingungen sowohl gegen die zu absorbierenden Gase als auch die Umweltbedingungen inert sind, geeignet. Im J. Electrochem. Soc. June 1957, Vol. 104, No. 6, Seiten 339-346 wird beispielsweise die Herstellung von anodischen Oxidschichten des Cr, Hf, Nb, V, Ta, Zr, Ti, Al beschrieben.

Als Träger für die Oxidschichten sind sowohl die Metalle selbst als auch Verbundwerkstoffe mit beliebigen anderen Trägermaterialien geeignet. Verfahren zur Herstellung dieser Werkstoffe gehören zum bekannten Stand der Technik.

Erfindungsgemäß bevorzugt werden nano-poröse metallische Materialien wie Aluminium/Aluminiumoxid (mit einer Porendichte von 10¹⁰-10¹²cm⁻² und einer Schichtdicke von bis zu 250 µm) oder Titandioxid, wie z.B. E3 der Firma Sachtleben GmbH, Duisburg.

Die Bildung der nanoporösen Aluminiumoxidschicht findet unter Gleich- oder Wechselstrom in Elektrolyten ungleich pH 7, wie z. B. Schwefelsäure, Oxalsäure, Phosphorsäure, Borsäure, Malonsäure und Chromsäure, statt. Das zu anodisierende Aluminium wird von dem jeweiligen Elektrolyten umspült und als Anode geschaltet. Als Kathode dient ein zu den jeweils verwendeten Elektrolyten inertes Metall. Durch die Variation von Spannung und Temperatur sowie die Wahl des Elektrolyten kann die Porengröße (Durchmesser der Pore), die Porendichte (Anzahl der Poren pro Quadratzentimeter) und die Härte (Bruchfestigkeit) der resultierenden Oxidschicht kontrolliert werden. Die Anodisierung schreitet so lange fort, bis der Stromfluss unterbrochen wird oder die Oxidschicht auf die maximal erreichbare Dicke angewachsen ist. Abhängig von der bei der Anodisierung vorherrschenden Temperatur bildet sich bei Temperaturen von 0 °C bis 5 °C eine harte Oxidschicht, bei Temperaturen über 5 °C eine weiche und biegsame Oxidschicht.

Es wird hierzu verwiesen auf die Veröffentlichungen von Parkhutik und Shershulski, J. Phys./D, 1992, Vol. 25, Seite 1258-1263; D. Hönicke, ALUMINIUM, 1989, Vol. 65, 11; auf die vollinhaltlich Bezug genommen wird.

Die Einlagerung des Aktivstoffs in der gebildeten porösen Oxidschicht bestehend aus hexagonalen Poren, kann sowohl mit einer Schmelze oder einer wässrigen Lösung des Aktivstoffs stattfinden. Die Einlagerung des Aktivstoffs erfolgt durch Behandlung der anodisierten Aluminiumbleche in 3 %iger Lösung von Zinkricinoleat mit Solubilisatoren oder in der Schmelze des Aktivstoffs bei 90 °C. Durch Erhöhung des Behandlungszeitraums kann die Menge des eingelagerten Aktivstoffs erhöht werden. Die prozentuale Menge des eingelagerten Aktivstoffs liegt im Allgemeinen nach 24 Stunden bei ca. 5 bis 7 %. Je nach Anodisierungsparameter kann der Massenanteil des eingelagerten Aktivstoffs nach oben oder unten abweichen. Nach anschließendem Trocknen ist der Aktivstoff fest mit der nanostrukturierten porösen Aluminiumoxidoberfläche verbunden.

Die Einlagerung des Aktivstoffs in nanostrukturierten Titandioxid-Formkörpern kann sowohl mit einer Schmelze oder einer wässrigen Lösung des Aktivstoffs stattfinden. Die Einlagerung des Aktivstoffs erfolgt durch Behandlung der nanostrukturierten Titandioxid-Formkörper in 3 %iger Lösung von Zinkricinoleat mit Solubilisatoren oder in der Schmelze des Aktivstoffs bei 90 °C. Durch Erhöhung des Behandlungszeitraums kann die Menge des eingelagerten Aktivstoffs erhöht werden. Die prozentuale Menge des eingelagerten Aktivstoffs liegt im Allgemeinen nach 24 Stunden bei ca. 3 bis 4 %. Nach anschließendem Trocknen ist der Aktivstoff fest mit den nanostrukturierten porösen Titandioxid-Formkörpern verbunden.

Zusätzlich können neben den aktiven Geruchsabsorbern, insbesondere Zinkricinoleat, bekannte Solubilisatoren und/oder Aktivatoren, insbesondere ein oder mehrere anionische Tenside, insbesondere Sulfosuccinate, Sulfosuccinamate und oder Sulfosuccinamide, Aminosäuren, insbesondere Arginin und Lysin, und/oder weitere desodorierende Wirkstoffe, wie z. B. quaternäre Ammoniumverbindungen oder Cyclodextrine, mitverwendet werden.

Die erfindungsgemäßen festen Geruchsabsorber können zur Desodorierung von geruchs- oder schadstoffbelasteten Gasen, wie sie in Klimaanlagen, Umluftanlagen, Haushaltsoberflächen, Abfallbehältern, Recyclingbehältern, Haushaltsgeräten auftreten können, Katzenstreu, Haustieren, Haustierschlafstätten, Vorhängen, Gardinen, Funktionstextilien, Autoinnenraumauskleidungen, in öffentlichen Bereichen mit hoher Personendichte wie z.B. in Warteräumen, Gaststätten, Flughäfen, Krankenhäusern, verwendet werden. Hier können die erfindungsgemäß modifizierten Aluminiumoberflächen ohne Flüssigkeit oder Aerosole in die Raumluft abzugeben die schlechten Gerüche reduzieren bzw. dekontaminieren.

### Beispiele:

### Beispiel 1:

### Verwendete Materialien:

- Solubilisiertes Zinkricinoleat (Tego^{®}Sorb A 30 der Firma Goldschmidt GmbH),
- anodisch oxidiertes Aluminium, hergestellt durch die anodische Oxidation von 99,99 % reinem Aluminium in 10 % Schwefelsäure während eines Zeitraumes von 24 Stunden bei einer Temperatur von 10 °C und einer Spannung von 20 V. Die eingesetzten porösen Oxidschichten weisen eine Porendichte von 10¹⁰ bis 10¹² cm⁻², eine Porengrößen von 10 bis 250 nm und eine Schichtdicke von bis zu 250 µm auf,
- Natriumsulfid.

In einem 100 ml Einhalsrundkolben mit Waschflaschenaufsatz wurde ein Milliliter einer 1 : 1 mit destilliertem Wasser verdünnten gesättigten Natriumsulfid-Lösung (pH 9 eingestellt) vorgelegt und sofort eines der zu untersuchenden trockenen anodisierten und mit Tego^{®}Sorb behandelten Al-Bleche dazugegeben. Die Belegung der anodisierten Bleche erfolgte durch Einlagerung des Aktivstoffs in einer 10 % Tego^{®}Sorb A 30-Lösung. Um den direkten Kontakt zwischen Blech und Natriumsulfid-Lösung zu vermeiden und nur eine Wechselwirkung zwischen Atmosphäre und anodisierten Oberfläche zu messen, wurden Plastikstücke als Abstandhalter unter das Blech gelegt.
Anschließend wurde nach bestimmten Zeiträumen die Atmosphäre des Kolbens durch ein Dräger-Multiwarn II-Messgerät abgezogen und der Gehalt von Schwefelwasserstoff bestimmt. Der Schwefelwasserstoffgehalt der Atmosphäre wurde bei mit Tego^{®}Sorb A 30 belegten Blechen sowie nur anodisierten Blechen und auch unbehandeltem Aluminium bestimmt.

Die erste Messung des Schwefelwasserstoffs in der Kolbenatmosphäre wurde nach zehn Minuten vorgenommen damit die Bildung einer homogenen Atmosphäre möglich war. Schon nach zehn Minuten war in der Atmosphäre des mit Tego^{®}Sorb belegten Bleches kein Schwefelwasserstoff mehr durch das Messgerät zu ermitteln. In den anderen Kolben mit den unbelegten oder nicht behandelten Aluminiumstücken war nach 24 Stunden noch ein Schwefelwasserstoffgehalt von mindestens 30 ppm festzustellen.

Im Folgenden sind die ermittelten Messwerte zur Übersicht tabellarisch aufgeführt.

**Tab. 1:**

| Vergleich anodisiertes und belegtes und nur anodisiertes Aluminiumblech | | |
|---|---|---|
| Zeit t [min] | Gehalt H₂S [ppm] anodisiertes Al mit Tego^{®}Sorb A 30 | Gehalt H₂S [ppm] anodisiertes, unbehandeltes Al-Blech |
| 0 | - | - |
| 10 | 0 | 15 |
| 50 | 0 | 22 |
| 100 | 0 | 24 |
| 120 | 0 | 14 |

**Tab. 2:**

| Vergleich anodisiertes und belegtes und nur anodisiertes Aluminiumblech | | |
|---|---|---|
| Zeit t [min] | Gehalt H₂S [ppm] Anodisiertes Al mit Tego^{®}Sorb A 30 | Gehalt H₂S [ppm] Anodisiertes, unbehandeltes Al-Blech |
| 0 | - | - |
| 15 | 0 | 24 |
| 45 | 0 | 26 |
| 120 | 0 | 33 |
| 24 h | 0 | > 100 |

**Tab. 3:**

| Vergleich anodisiertes nicht belegtes und unbehandeltes Aluminiumblech | | |
|---|---|---|
| Zeit t [min] | Gehalt H₂S [ppm] Anodisiertes, unbehandeltes Al-Blech | Gehalt H₂S [ppm] Al-Blech unbehandelt |
| 0 | - | - |
| 10 | 31 | 24 |
| 40 | 31 | 32 |
| 70 | 29 | 33 |
| 120 | 30 | 36 |
| 24 h | 27 | 29 |

Die in Tab. 1, 2 und 3 verglichenen Bleche wurden unter identischen Bedingungen, bei 10 °C und 20 V, in 10 % Schwefelsäure anodisiert.

### Ergebnisse:

Die Ergebnisse der Tabellen 1 bis 3 zeigen deutlich, dass eine effektive Entfernung von Schwefelwasserstoff nur bei den anodisierten Aluminium-Blechen in Verbindung mit eingelagertem Tego^{®}Sorb zu erkennen ist. Weder resultiert eine Verminderung oder Entfernung des Geruchs- und Schadstoffs Schwefelwasserstoff bei Verwendung von unbehandeltem Aluminium, noch bei Verwendung von anodisiertem Aluminium. Somit ist die Wirksamkeit von Tego^{®}Sorb in anodisch hergestellten porösen Aluminiumoxidschichten bewiesen.

### Beispiel 2:

### Verwendete Materialien:

- Solubilisiertes Zinkricinoleat (Tego^{®}Sorb A 30 der Firma Goldschmidt GmbH),
- Anodisch oxidiertes Aluminium, hergestellt durch die anodische Oxidation von 99,99 % reinem Aluminium in 10 % Schwefelsäure während eines Zeitraumes von 24 Stunden bei einer Temperatur von 10 °C und einer Spannung von 20 V. Die eingesetzten porösen Oxidschichten weisen eine Porendichte von 10¹⁰ bis 10¹² cm⁻², eine Porengrößen von 10 bis 250 nm und eine Schichtdicke von bis zu 250 µm auf,
- Ammoniak 25 %ig.

Ein Milliliter einer 1 : 20 bzw. 1 : 10-Verdünnung von 25 % Ammoniumhydroxid wird jeweils mit einem anodisierten, mit Tego^{®}Sorb A 30 belegten, Aluminiumblech in ein Schraubdeckelgefäß gegeben. Zwei weitere Schraubdeckelgefäße nur mit den Ammoniak-Verdünnungen und ohne Aluminiumblech dienten als Blindprobe. Um den direkten Kontakt zwischen Blech und Ammoniak zu vermeiden und nur eine Wechselwirkung zwischen Atmosphäre und anodisierten Oberfläche zu messen, wurden Plastikstücke als Abstandhalter unter das Blech gelegt.

Nach einer Dauer von 24 h ist in den Rundkolben mit Tego^{®}Sorb A 30 belegten Aluminiumblechen eine Verringerung des pH-Wertes von pH 11 nach pH 10 zu erkennen, während in den anderen Kolben noch ein pH-Wert von 11 besteht.
Der resultierende pH-Wert der wässrigen Ammoniak-Phase wurde mit Hilfe von Universalindikator-Stäbchen der Firma Merck (pH 0 bis 14) ermittelt.

### Beispiel 3:

### Verwendete Materialien:

- Solubilisiertes Zinkricinoleat (Handelsprodukt Tego^{®}Sorb 80 der Firma Goldschmidt GmbH),
- Titandioxid (Titandioxid E3 der Firma Sachtleben Chemie GmbH),
- Ammoniak 25 %ig.

In ein Schraubdeckelgefäß wurden jeweils 4 g mit Tego^{®}Sorb 80 belegtes Titandioxid und 1 ml einer 1 : 10 und 1 : 20 verdünnten 25 %igen Ammoniak-Lösung vorgelegt. Zwei weitere Schraubdeckelgefäße nur mit den Ammoniak-Verdünnungen und ohne Titandioxid dienten als Blindprobe.

Damit keine Berührungspunkte zwischen trockenem Titandioxid und Flüssigkeit resultierten, wurde das Titandioxid innerhalb des Schraubdeckelgefäßes durch einen kleinen Behälter separiert.

Nach 18 h erfolgte eine pH-Messung in den vier Gefäßen. Die beiden ohne Titandioxid ausgestatteten Schraubdeckelgefäße zeigten einen pH-Wert von 10 und stechender Ammoniak-Geruch war noch zu erkennen.

Die beiden mit Titandioxid ausgestatteten Schraubdeckelgefäße zeigten eine Verringerung des pH-Wertes nur über die Gasphase. Der resultierende pH-Wert bei der 1 : 10-Verdünnung lag nach 18 h bei pH 8 bis 9 und bei der 1 : 20-Verdünnung bei pH 7 bis 8.

Um die Ergebnisse auch optisch sichtbar zu machen, wurden in die Schraubdeckelgefäße jeweils pH-Universalindikator-Papierstreifen gehängt. Der resultierende pH-Wert wurde mit Hilfe von Universalindikator-Stäbchen der Firma Merck (pH 0 bis 14) ermittelt.

### Ergebnisse:

Die Resultate aus Beispiel 2 und 3 zeigen deutlich eine Verminderung des pH-Wertes der Gasphase und vor allem der Flüssigkeit in den Schraubdeckelgefäßen bzw. Einhalsrundkolben. Die Ergebnisse zeigen deutlich, dass mit Tego®Sorb belegte Titandioxid-Formkörper bzw. anodisierte Aluminiumbleche über die Gasphase zu einem Abbau von Ammoniak führen.

### Beispiel 4:

### Verwendete Materialien:

- Solubilisiertes Zinkricinoleat (Tego^{®}Sorb A 30 der Firma Goldschmidt GmbH),
- Titandioxid (Titandioxid E3 der Firma Sachtleben Chemie GmbH),
- Natriumsulfid.

In einem 100 ml Einhalsrundkolben mit Waschflaschenaüfsatz wurde ein Milliliter einer 1 : 1 mit destilliertem Wasser verdünnten gesättigten Natriumsulfid-Lösung (pH 9 eingestellt) vorgelegt und sofort die zu untersuchenden mit Tego^{®}Sorb A 30 belegten Titandioxid-Formkörper dazugegeben. Die Belegung der Titandioxid-Formkörper erfolgte durch Einlagerung des Aktivstoffs in einer 10 % Tego^{®}Sorb A 30-Lösung. Um den direkten Kontakt zwischen Blech und Natriumsulfid-Lösung zu vermeiden und nur eine Wechselwirkung zwischen Atmosphäre und anodisierten Oberfläche zu messen, wurden Plastikstücke als Abstandhalter unter das Titandioxid gelegt.

Anschließend wurde nach bestimmten Zeiträumen die Atmosphäre des Kolbens durch ein Dräger-Multiwarn II-Messgerät abgezogen und der Gehalt von Schwefelwasserstoff bestimmt.

Der Schwefelwasserstoffgehalt der Atmosphäre wurde bei mit Tego^{®}Sorb A 30 belegtem Titandioxid sowie nicht belegtem Titandioxid und nur Natriumsulfid-Lösung als Blindprobe bestimmt.

Die erste Messung des Schwefelwasserstoffs in der Kolbenatmosphäre wurde nach fünf Minuten vorgenommen damit die Bildung einer homogenen Atmosphäre möglich war. Schon nach fünf Minuten war in der Atmosphäre des mit Tego^{®}Sorb A 30 belegten Titandioxid-Formkörper kein Schwefelwasserstoff mehr durch das Messgerät zu ermitteln. In den anderen Kolben mit den unbelegten Titandioxid-Formkörpern bzw. der Blindprobe war auch nach 24 Stunden noch Schwefelwasserstoff festzustellen.

Im Folgenden sind die ermittelten Messwerte zur Übersicht tabellarisch aufgeführt.

**Tab. 4:**

| Vergleich belegtes/nicht belegtes Titandioxid (Sachtleben) | | | |
|---|---|---|---|
| Zeit t [min] | Gehalt H₂S [ppm] Titandioxid unbehandelt | Gehalt H₂S [ppm] mit Tego^{®}Sorb A 30 belegtes Titandioxid | Gehalt H₂S [ppm] Blindprobe (ohne Titandioxid) |
| 0 | - | - | - |
| 5 | 14 | 0 | 24 |
| 30 | 15 | 0 | 24 |
| 60 | 12 | 0 | 15 |
| 120 | 10 | 0 | 10 |
| 210 | 8 | 0 | 6 |
| 270 | 10 | 0 | 12 |
| 24 h | 3 | 0 | 12 |

### Ergebnisse:

Die Ergebnisse der Tabelle 4 zeigen deutlich, dass eine effektive Entfernung von Schwefelwasserstoff nur bei den mit Tego^{®}Sorb behandelten Titandioxid-Formkörpern zu erkennen ist. Es resultiert keine Verminderung oder Entfernung des Geruchs- und Schadstoffs Schwefelwasserstoff bei Verwendung von unbehandelten Titandioxid-Formkörpern.

## Patentansprüche

1. Feste Geruchsabsorber enthaltend einen festen Träger mit nanostrukturierter Oberfläche und darin eingelagertem aktiven Geruchsabsorber.

2. Feste Geruchsabsorber gemäß Anspruch 1 **dadurch gekennzeichnet, dass** als festes Trägermaterial nanoporöse Materialien verwendet werden die fähig sind, Wasser an der Oberfläche zu fixieren.

3. Feste Geruchsabsorber gemäß Anspruch 1 und/oder 2 **dadurch gekennzeichnet, dass** als festes Trägermaterial nanoporöse Metalloxide verwendet werden.

4. Feste Geruchsabsorber gemäß mindestens einem der Ansprüche 1 bis 3 **dadurch gekennzeichnet, dass** der aktive Geruchsabsorber mindestens eine Verbindung ausgesucht aus der Gruppe von Metallsalzen einer unverzweigten oder verzweigten, ungesättigten oder gesättigten, ein oder mehrfach hydroxylierten Fettsäure mit mindestens 16 Kohlenstoffatomen und/oder einer Harzsäure mit Ausnahme der Alkalimetallsalze sowie aus beliebigen Mischungen dieser Salze mit sich selbst enthält.

5. Feste Geruchsabsorber gemäß Anspruch 4 **dadurch gekennzeichnet, dass** die Metallsalze Zinksalze sind.

6. Feste Geruchsabsorber gemäß Anspruch 5 **dadurch gekennzeichnet, dass** die Zinksalze in Kombinationen mit Zinksalzen der Abietinsäure oder mit Zinksalzen anderer gesättigter oder ungesättigter hydroxylierter Fettsäuren mit 16 und mehr C-Atomen eingesetzt werden.

7. Feste Geruchsabsorber gemäß Anspruch 5 **dadurch gekennzeichnet, dass** der aktive Geruchsabsorber Zinksalze von Fettsäuren, vorzugsweise das Zinksalz der Ricinolsäure enthält.

8. Feste Geruchsabsorber gemäß mindestens einem der Ansprüche 1 bis 7 **dadurch gekennzeichnet, dass** der aktive Geruchsabsorber mit bekannten Solubilisatoren und/oder Aktivatoren, insbesondere einem oder mehreren anionischen Tensiden, Aminosäuren, insbesondere Arginin und Lysin, und/oder mit weiteren desodorierenden Wirkstoffen, wie quaternäre Ammoniumverbindungen oder Cyclodextrinen, kombiniert wird.

9. Verwendung der Geruchsabsorber gemäß mindestens einem der Ansprüche 1 bis 8 zur Desodorierung von geruchs-oder schadstoffbelasteten Gasen.
